# EUROPEAN PATENT SPECIFICATION

(11) **EP 1 614 406 B1**
(45) Date of publication and mention of the grant of the patent: **19.09.2012**
(21) Application number: 04724171.6
(22) Date of filing: 29.03.2004
(51) Int. Cl.: A61F 13/475, A61F 13/471

(54) **ABSORBING PAD FOR MALE**
SAUGEINLAGE FÜR MÄNNER
TAMPON ABSORBANT POUR HOMMES

(30) Priority: 31.03.2003 JP 2003096159; 30.05.2003 JP 2003155601; 30.09.2003 JP 2003341868
(43) Date of publication of application: 11.01.2006
(73) Proprietor: DAIO PAPER CORPORATION, Shikokuchuo-shi, Ehime 799-0492 (JP)
(72) Inventor: SHIOMI, Takeyuki, ELLEAIR PAPER TECH. CO., LTD., Sakura-shi, Tochigi 329-1411 (JP); ISHIDA, Tomoaki, ELLEAIR PAPER TECH. CO., LTD., Sakura-shi, Tochigi 329-1411 (JP)
(74) Representative: Held, Stephan
(86) International application number: PCT/JP2004/004444
(87) International publication number: WO 2004/087027

(56) References cited:
- EP-A- 0 787 473
- JP-A- 9 506 280
- JP-A- 11 267 145
- JP-A- 2001 224 617
- JP-A- 2002 509 462
- JP-A- 2003 204 980

## Description

### TECHNICAL FIELD

The present invention relates to an absorbent pad for men configured so that a back sheet, an absorbent member, and a liquid permeable top sheet are superimposed from below in this order, peripheral ends of the back sheet and the top sheet are coupled to each other, a gather sheet is provided on the top sheet to thereby form a penile opening for inserting a penis, and so that the inserted penis is held.

The present invention relates to an absorbent pad for men for a bed-ridden male, a male who suffers from incontinence, or the like to wear the pad together with a disposable diaper or through briefs. More specifically, the present invention relates to an absorbent pad for men excellent in leakage prevention effect and easy to wear.

### BACKGROUND ART

A conventional absorbent pad for men is a rectangular pad including a liquid permeable top sheet, a liquid impermeable back sheet equal in shape to the top sheet, and an absorbent member arranged between the top and back sheets. When in use, the rectangular absorbent pad for men is wadded up generally into a conical shape around one corner, a male user wears the pad with the pad wound around the penis, and an outer periphery of the pad is fixed by a tape or the like.

The absorbent pad for men of this type has, however, the following disadvantages. Since the pad is used by directly winding the pad around the penis, the male user feels something strange or feels tightened up in the penis and the pad is difficult to use. If the absorbent pad is wound around the penis so that the male user does not feel anything strange or tightened up, the pad is prone to be detached from the penis and leakage of urine occurs.

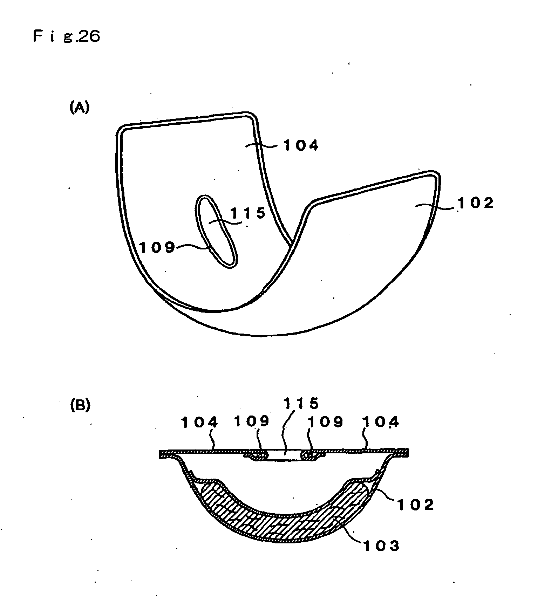

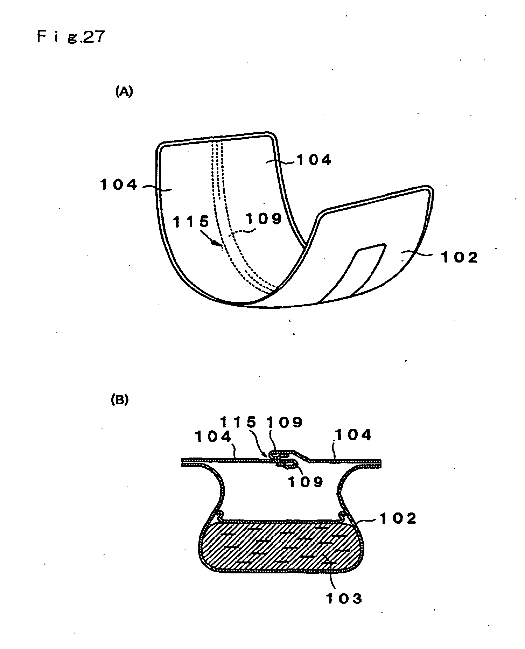

To address the disadvantages, an absorbent pad for men as shown in Figs. 26(A), 26(B), 27(A), and 27(B) is disclosed (see, for example, Kohyo (National Publication of Translated Version) No. 2002-509462). Namely, the absorbent pad includes a liquid permeable top sheet 101, a liquid impermeable back sheet 102, an absorbent member 103 arranged between the top and back sheets 101 and 102, and a liquid impermeable gather sheet 104 arranged on the top sheet 101. The gather sheet 104 includes an opening 115 and an elastic member 109 is arranged around the opening 115. The wear's genitals are inserted into the opening 115 of the absorbent pad for men and held by the pad, and excrement is absorbed by the absorbent member 103 through the top sheet 101. By so configuring, it is unnecessary to attach the pad to the wearer by directly winding the pad around the penis, thereby making it possible to lessen the disadvantage in that the wearer feels something strange.

Meanwhile, there is proposed another absorbent pad for men including a liquid permeable top sheet, a liquid impermeable back sheet, and an absorbent member arranged between the top and back sheets (as disclosed in, for example, Japanese Patent Application Laid-Open No. 2002-52036). Namely, a sack-like absorbent pad for men is configured so that a rectangular absorbent pad is folded into three in a longitudinal direction and so that parts at about one-third positions of ends in the longitudinal direction and a right angle direction are folded back and fixed.

The former absorbent pad for men has, however, the following disadvantages. When the wearer lies on his back, the urine is not completely absorbed by the absorbent member 103 but arrives at the gather sheet 104 provided on wearer's belly side. Since the gather sheet 104 is liquid impermeable, the urine is not absorbed by the gather sheet 104, flows on a surface of the gather sheet 104, arrives at the opening 115, and leaks to the outside. As a result, the garment of the wearer or a paper diaper used as well as this pad is contaminated.

The latter absorbent pad for men has the following disadvantage. Since the pad fails to provide a structure of fixing the male genitals, the pad is detached to cause leakage of urine.

Other absorbent pads are disclosed, e.g. in US 5,558,659 and WO 98/48753A1.

### DISCLOSURE OF THE INVENTION

It is, therefore, an object of the present invention to provide a leakage-free absorbent pad for men.

To solve the disadvantages, the invention according to claim 1 is an absorbent pad for men comprising: a liquid permeable top sheet; a back sheet; an absorbent member arranged between the top sheet and the back sheet; and a pair of liquid impermeable gather sheets facing each other on the top sheet, wherein a liquid permeable auxiliary sheet is provided to extend from a side of one of the gather sheets toward the other one of the gather sheets between the top sheet and the gather sheets, and an opening for inserting male genitals is formed by the auxiliary sheet and the other gather sheet.

According to the invention according to claim 1, the male genitals are held between the auxiliary sheet and the top sheet. Due to this, even if the wearer excretes liquefied excrement while lying on his back and the liquefied excrement is not completely absorbed by the absorbent member, the auxiliary sheet abates the force of the liquefied excrement and diffuses the liquefied excrement. This can prevent the liquefied excrement that flows without being absorbed by the absorbent member from reaching the opening through the gather sheet and leaking outside. It is, therefore, possible to provide the absorbent pad for men which can prevent leakage. Besides, the wearer does not feel something strange when wearing the absorbent pad for men and can comfortably wear the absorbent pad for men.

The invention according to claim 2 is the absorbent pad for men according to claim 1, wherein the auxiliary sheet is provided integrally with the top sheet.

According to the invention according to claim 2, the auxiliary sheet can be provided simultaneously with the top sheet in the step of providing the top sheet. It is, therefore, possible to simplify steps and efficiently manufacture the absorbent pad for men.

The invention according to claim 3 is the absorbent pad for men according to claim 1, wherein the auxiliary sheet is provided separately from the top sheet.

According to the invention according to claim 3, the auxiliary sheet can be configured by the material higher in diffusing property than the top sheet. It is, therefore, possible to provide the absorbent pad for men that can further prevent leakage.

The invention according to claim 4 is the absorbent pad for men according to any one of claims 1 to 3, wherein the back sheet is liquid impermeable.

According to the invention according to claim 4, the liquefied excrement does not leak from the back sheet. It is, therefore, possible to provide the absorbent pad for men that can prevent leakage.

The invention according to claim 5 is the absorbent pad for men according to any one of claims 1 to 3, wherein the back sheet is liquid permeable.

According to the invention according to claim 5, if the absorbent pad for men is used while being superimposed on a used surface of a paper diaper, the liquefied excrement that is not completely absorbed by the absorbent pad for men can be permeated and absorbed by an absorbent member included in the paper diaper. It is, therefore, possible to provide the absorbent pad for men that can prevent excrement from leaking from the paper diaper even if the excrement is large in amount. The invention according to claim 6 is the absorbent pad for men according to any one of claims 1 to 5, wherein the absorbent member is a rectangle with corners notched.

According to the invention according to claim 6, it is possible to provide the absorbent pad for men that can be fit to the hip joint of the wearer and that can facilitate the wearer's motion.

According to the invention, an elastic member is provided on a tip end of the auxiliary sheet.

Thus, it is possible to provide the absorbent pad for men that can ensure that the auxiliary sheet is fit to the male genitals and that can further prevent leakage.

The invention according to claim 7 is the absorbent pad for men according to any one of claims 1 to 6, wherein the opening formed by a tip end of the auxiliary sheet and a tip end of the other gather sheet is unevenly distributed toward one of the gather sheets.

According to a preferred embodiment, if the male genitals are inserted into the absorbent pad for men, then the tip end of the penis can be held near the center of the absorbent member, and the liquefied excrement can be effectively absorbed by the absorbent member.

The invention further claims for an absorbent pad for men, wherein the tip end of the other gather sheet and the tip end of the auxiliary sheet are generally at the same position.

Therefore, the auxiliary sheet effectively closes the opening, so that it is possible to further prevent the liquefied excrement from leaking from within the absorbent pad for men to the outside.

According to the absorbent pad for men, the tip end of the one gather sheet and the tip end of the auxiliary sheet can be generally at the same position.

According to a preferred embodiment, the male genitals inserted and held into the auxiliary sheet are also held in one of the water-repellent gather sheets. It is, therefore, possible to provide the absorbent pad for men that can further prevent leakage.

In the following, several alternatives of absorbent pads which are not part of the present invention are disclosed:

According to a first alternative an absorbent pad for men comprises: a liquid permeable top sheet; a back sheet; an absorbent member arranged between the top sheet and the back sheet; and a gather sheet located at least partially on the top sheet, an opening for inserting male genitals being formed by the gather sheet; wherein a urination pocket is formed by folding back the absorbent pad for men with the top sheet located inside, and a liquid passage portion is provided by providing a gap between the top sheet and the gather sheet in a folded portion within the urination pocket.

According to the first alternative, in the absorbent pad for men in which the male genitals are fixedly inserted from the opening formed by the gather sheet, the urine excreted from the male genitals at a breath can be smoothly absorbed by the absorbent member within the urination pocket. It is, therefore, possible to provide the absorbent pad for men that can prevent leakage.

According to a second alternative the absorbent pad for men comprises: a liquid impermeable first gather sheet located at least partially on the top sheet; and a liquid impermeable second gather sheet located at least partially on the top sheet, wherein an opening for inserting the male genitals is formed by the first gather sheet and the second gather sheet.

According to the second alternative, in the absorbent pad for men in which the male genitals are fixedly inserted from the opening formed by the gather sheet, the urine excreted from the male genitals at a breath can be received in the urination pocket and smoothly absorbed by the absorbent member on the folded side through the liquid passage portion provided between the top sheet and the second gather sheet. In addition, the liquid impermeable second gather sheet provided to extend from the end of the absorbent member on the folded side can prevent the urine from running over from within the urination pocket. It is, therefore, possible to provide the absorbent pad for men that can prevent leakage.

A third alterntaive is the absorbent pad for men according the second alternative, wherein a urination pocket is formed by folding back the absorbent pad for men along a tip end direction of the first gather sheet and the second gather sheet with the top sheet located inside, and a liquid passage portion is provided by providing a gap between the top sheet and the second gather sheet.

According to the third alternative, the excreted urine goes round between the liquid impermeable second gather sheet and the top sheet, the urine is smoothly absorbed by the absorbent member on the folded side, and the second gather sheet prevents the urine from leaking outside.

The fourth alternative is an absorbent pad for men comprising: a liquid permeable top sheet; a back sheet, an absorbent member arranged between the top sheet and the back sheet; and a gather sheet located at least partially on the top sheet, an opening for inserting male genitals being formed by the gather sheet, wherein a urination pocket is formed by folding back the absorbent pad for men with the top sheet located inside, and a liquid impermeable auxiliary gather sheet for preventing leakage of urine staying in the urination pocket is provided on the top sheet on a folded side.

According to the fourth alternative, in the male in the absorbent pad for men in which the male genitals are fixedly inserted from the opening formed by the gather sheet, the urine excreted from the male genitals at a breath can be smoothly absorbed by the absorbent member within the urination pocket. In addition, the liquid impermeable auxiliary gather sheet provided to extend from the end of the absorbent member on the folded side can prevent the urine from running over from within the urination pocket. It is, therefore, possible to provide the absorbent pad for men that can prevent leakage.

The fifth alternative is the absorbent pad for men according to the fourth alternative, comprising: a liquid impermeable first gather sheet located at least partially on the top sheet; and a liquid impermeable second gather sheet located at least partially on the top sheet, wherein an opening for inserting the male genitals is formed by the first gather sheet and the second gather sheet.

According to the fifth alternative, in the absorbent pad for men in which the male genitals are fixedly inserted from the opening formed between the first gather sheet and the second gather sheet, the urine excreted from the male genitals at a breath can be received in the urination pocket and smoothly absorbed by the absorbent member on the folded side through the liquid passage portion provided between the top sheet and the second gather sheet. In addition, the liquid impermeable second gather sheet provided to extend from the end of the absorbent member on the folded side can prevent the urine from running over from within the urination pocket. It is, therefore, possible to provide the absorbent pad for men that can prevent leakage.

The sixth alternative is the absorbent pad for men according to the fifth alternative, wherein a urination pocket is formed by folding back the absorbent pad for men along a tip end direction of the first gather sheet and the second gather sheet with the top sheet located inside, and a liquid impermeable auxiliary gather sheet for preventing the leakage of urine staying in the urination pocket is provided on the top sheet on a folded side.

According to the sixth alternative, since the auxiliary gather sheet is provided on the top sheet on the folded side, it is possible to further prevent the urine from leaking from within the urination pocket.

The seventh alternative is the absorbent pad for men according to the fifth or sixth alternative, wherein the second gather sheet is formed along a part of the urination pocket.

According to the seventh alternative, it is possible to provide the absorbent pad for men at low cost without making the second gather sheet long.

The eighth alternative is the absorbent pad for men according to the fourth alternative, comprising: a liquid impermeable first gather sheet located at least partially on the top sheet; and a liquid impermeable second gather sheet located at least partially on the top sheet, wherein an opening for inserting the male genitals is formed by the first gather sheet and the second gather sheet.

According to the eighth alternative, if the absorbent pad for men is used while male genitals being inserted between the liquid permeable top sheet and the liquid permeable second gather sheet, the second gather sheet abates the force of the urine and enables the urine to be permeated by the top sheet during urination. It is, therefore, possible to prevent the leakage of urine from the urination pocket.

The ninth alternative is the absorbent pad for men according to the fifth alternative, wherein a urination pocket is formed by folding back the absorbent pad for men along a tip end direction of the first gather sheet and the second gather sheet with the top sheet located inside, and a liquid impermeable auxiliary gather sheet for preventing the leakage of the urine staying in the urination pocket is provided on the top sheet on the folded side.

According to the ninth alternative, since the auxiliary gather sheet is provided on the top sheet on the folded side, it is possible to further prevent leakage of urine from within the urination pocket.

The tenth alternative is the absorbent pad for men according to the ninth alternative, wherein the second gather sheet is provided integrally with the top sheet.

According to the tenth alternative, the second gather sheet can be provided simultaneously with the top sheet in the step of providing the top sheet. It is, therefore, possible to simplify steps and efficiently manufacture the absorbent pad for men.

The eleventh alternative is the absorbent pad for men according to any one of the first to tenth alternatives, wherein the back sheet is liquid impermeable.

According to the eleventh alternative, the absorbent pad for men can prevent the urine absorbed by the absorbent member from being permeated outside from the back sheet. The unfolded paper diaper, briefs type paper diaper, briefs type underwear or the like which the wearer wears outside the absorbent pad for men is not contaminated by the urine.

The twelve alternative is the absorbent pad for men according to any one of the first to tenth alternatives, wherein the back sheet is liquid permeable.

According to the twelve alternative, if the absorbent pad for men is used together with a paper diaper, the urine oozing out from the back sheet is absorbed by an absorbent member of the paper diaper. A urine allowable absorption capacity is, increased, and the absorbent pad for men can be used for a long period of time.

The thirteenth alternative is the absorbent pad for men according to any one of the first to twelve alternatives, wherein the opening formed by a tip end of the first gather sheet and a tip end of the second gather sheet is unevenly distributed toward the first gather sheet.

According to the thirteenth alternative, if the male genitals are inserted into the absorbent pad for men, then the tip end of the penis can be held near the center of the absorbent member, and the liquefied excrement can be effectively absorbed by the absorbent member.

The fourteenth alternative is the absorbent pad for men according to any one of the first to thirteenth alternatives, wherein the tip end of the first gather sheet and the tip end of the second gather sheet are generally at the same position.

According to the fourteenth alternative, the first gather sheet and the second gather sheet can effectively closes the opening, so that it is possible to further prevent the urine from leaking outside from within the urination pad.

The fifteenth alternative is the absorbent pad for men according to any one of the first to thirteenth alternatives, wherein the tip end of the first gather sheet and the tip end of the second sheet are overlapped with each other.

According to the fifteenth alternative, the first gather sheet and the second gather sheet can further effectively close the opening, so that it is possible to far further prevent the urine from leaking outside from within the urination pad.

The sixteenth alternative is the absorbent pad for men according to any one of the first to fifteenth alternatives, wherein the folded portion within the urination pocket is not overlapped with the opening for inserting the male genitals.

According to the sixteenth alternative, the absorbent pad for men can be effectively used.

The seventeenth alternative is an absorbent pad for men configured so that a back sheet, an absorbent member, and a liquid permeable top sheet are superimposed from below in this order, peripheral edges of the back sheet are bonded to peripheral edges of the top sheet to form bonded portions, respectively, a gather sheet having an opening is provided on the top sheet, and so that male genitals are inserted from the opening of the gather sheet and held by the absorbent pad for men, wherein a liquid impermeable first gather sheet is provided from one end out of front and rear end of the top sheet in a longitudinal direction toward a central portion of the top sheet, a second gather sheet, a second gather sheet is formed by angle-folding the top sheet from a direction of the other end toward the central portion, the opening is formed to be opened when in use to overlap tip ends of the first gather sheet and the second gather sheet with each other so as to be able to insert the male genitals therefrom, a water-repellent strip is provided on each side of the absorbent member on the top sheet in the longitudinal direction of the top sheet, and wherein the absorbent pad for men is folded back in the longitudinal direction to further bond the bonded portions by the absorbent pad for men as well as the first gather sheet and the second gather sheet, a pocket being formed on the each side of the absorbent member.

According to the seventeenth alternative, the liquid impermeable gather sheet is provided from one of the front and rear ends of the top sheet in the longitudinal direction toward the central portion of the top sheet, and the top sheet is folded back into a crest shape from the other end toward the central portion thereof, thereby forming the second gather sheet. The opening is formed by superimposing tip ends of the first and second gather sheets. The water-repellent strip is provided on each side of the absorbent member on the top sheet in the longitudinal direction thereof. The absorbent pad for men is folded back in the longitudinal direction to further bond the bonded portions, and the pocket is formed on the each side of the absorbent member. Due to this, if the wearer urinates while lying on his side, then the urinate is not permeated by up to the both sides of the absorbent member, and the excreted urine is made temporarily stay in the pocket. It is, therefore, possible to provide the absorbent pad for men that can prevent leakage of urine. In addition, since the top sheet and the second gather sheet are provided integrally with each other, it is possible to provide the absorbent pad for men that can prevent leakage of urine even if the wearer urinates while lying on his side with simple configuration.

The eighteenth alternative is an absorbent pad for men configured so that a back sheet, an absorbent member, and a liquid permeable top sheet are superimposed from below in this order, peripheral edges of the back sheet are bonded to peripheral edges of the top sheet to form bonded portions, respectively, a gather sheet having an opening is provided on the top sheet, and so that male genitals are inserted from the opening of the gather sheet and held by the absorbent pad for men, wherein a liquid impermeable first gather sheet is provided from one end out of front and rear end of the top sheet in a longitudinal direction toward a central portion of the top sheet, and a second gather sheet extending from a direction of the other end toward the central portion of the top sheet is provided on the top sheet, and the opening is formed to be opened when in use to overlap tip ends of the first gather sheet and the second gather sheet with each other so as to be able to insert the male genitals therefrom, a water-repellent strip is provided on each side of the absorbent member in the longitudinal direction of the top sheet, and wherein the absorbent pad for men is folded back in the longitudinal direction to further bond the bonded portions, a pocket being formed on the each side of the absorbent member.

According to the eighteenth alternative, the liquid impermeable first gather sheet is provided from one of the front and rear ends of the top sheet in the longitudinal direction toward the central portion of the top sheet, and the second gather sheet extending from the other end direction toward the central portion of the top sheet in the longitudinal direction is provided on the top sheet. The opening is formed by superimposing the tip ends of the first and second gather sheets, and the water-repellent strip is provided on each side of the absorbent member on the top sheet in the longitudinal direction thereof. The absorbent pad for men is folded back in the longitudinal direction to further bond the bonded portions, and the pocket is formed on the each side of the absorbent member. It is, therefore, possible to provide the absorbent pad for men that can prevent the urine from being permeated by up to the both sides of the absorbent member if the wearer urinates while lying on his side, that can cause the excreted urine to temporarily stay in the pocket, and that can prevent leakage of urine even if the wearer urinates while lying on his side.

The nineteenth alternative is an absorbent pad for men configured so that a back sheet, an absorbent member, and a liquid permeable top sheet are superimposed from below in this order, a liquid impermeable water-repellent sheet is provided to cover both sides of the absorbent member and peripheral edges of the back sheet are bonded to peripheral edges of the water-repellent sheet to form bonded portions, respectively, a gather sheet having an opening is provided on the top sheet, and so that male genitals are inserted from the opening of the gather sheet and held by the absorbent pad for men, wherein a liquid impermeable first gather sheet is provided from one end out of front and rear end of the top sheet in a longitudinal direction toward a central portion of the top sheet, and a second gather sheet is formed by angle-folding the top sheet from a direction of the other end toward the central portion, the opening is formed to be opened when in use to overlap tip ends of the first gather sheet and the second gather sheet with each other so as to be able to insert the male genitals therefrom, and the absorbent pad for men is folded in the longitudinal direction to further bond the bonded portions by the absorbent pad for men as well as the first gather sheet and the second gather sheet, a pocket being formed on each of the both sides of the absorbent member.

According to the nineteenth alternative, the liquid impermeable first gather sheet is provided from one of the front and rear ends of the top sheet in the longitudinal direction toward the central portion of the top sheet, and the second gather sheet extending from the other end direction toward the central portion of the top sheet in the longitudinal direction is provided on the top sheet. The opening is formed by superimposing the tip ends of the first and second gather sheets, and the water-repellent strip is provided on each side of the absorbent member on the top sheet in the longitudinal direction thereof. The absorbent pad for men is folded back in the longitudinal direction to further bond the bonded portions, and the pocket is formed on the each side of the absorbent member. Due to this, the bonded portions consist only of the water-repellent material. It is, therefore, possible to far further prevent leakage of urine from the sides of the absorbent pad for men. Thus, the absorbent pad for men that can farther prevent leakage of urine, that can prevent the urine from being permeated by up to the both sides of the absorbent member if the wearer urinates while lying on his side, and that can cause the excreted urine to temporarily stay in the pocket. In addition, since the top sheet and the second gather sheet are provided integrally with each other, it is possible to provide the absorbent pad for men that can prevent leakage of urine even if the wearer urinates while lying on his side with simple configuration.

The twentieth alternative is an absorbent pad for men configured so that a back sheet, an absorbent member, and a liquid permeable top sheet are superimposed from below in this order, a liquid impermeable water-repellent sheet is provided to cover both sides of the absorbent member and peripheral edges of the back sheet are bonded to peripheral edges of the water-repellent sheet to form bonded portions, respectively, a gather sheet having an opening is provided on the top sheet, and so that male genitals are inserted from the opening of the gather sheet and held by the absorbent pad for men, wherein a liquid impermeable first gather sheet is provided from one end out of front and rear end of the top sheet in a longitudinal direction toward a central portion of the top sheet, a second gather sheet extending from a direction of the other end toward the central portion of the top sheet in the longitudinal direction is provided on the top sheet, and the opening is formed to be opened when in use to overlap tip ends of the first gather sheet and the second gather sheet with each other so as to be able to insert the male genitals therefrom, and wherein the absorbent pad for men is folded in the longitudinal direction to further bond the bonded portions by the absorbent pad for men as well as the first gather sheet and the second gather sheet, a pocket being formed on each of the both sides of the absorbent member.

According to the twentieth alternative, the liquid impermeable first gather sheet is provided from one of the front and rear ends of the top sheet in the longitudinal direction toward the central portion of the top sheet, and the second gather sheet extending from the other end direction toward the central portion of the top sheet in the longitudinal direction is provided on the top sheet. The opening is formed by superimposing the tip ends of the first and second gather sheets, and the water-repellent strip is provided on each side of the absorbent member on the top sheet in the longitudinal direction thereof. The absorbent pad for men is folded back in the longitudinal direction to further bond the bonded portions, and the pocket is formed on the each side of the absorbent member. Due to this, the bonded portions consist only of the water-repellent material. It is possible to far further prevent leakage of urine from the sides of the absorbent pad for men, prevent the urine from being permeated by up to the both sides of the absorbent member if the wearer urinates while lying on his side, and cause the excreted urine to temporarily stay in the pocket. It is, therefore, possible to provide the absorbent pad for men that can prevent leakage of urine even if the wearer urinates while lying on his side.

The twenty-first alternative is the absorbent pad for men according to the seventeenth or eighteenth alternative, wherein the water-repellent strip is a water-repellent sheet.

According to the twenty-first alternative, since the water-repellent strip is a water-repellent sheet, it is possible to easily manufacture the absorbent pad for men.

The twenty-second alternative is the absorbent pad for men according to the seventeenth or eighteenth alternatives, wherein the water-repellent strip is formed by subjecting the top sheet to water repellent finishing.

According to the twenty-second alternative, since the water-repellent strip is formed by subjecting the top sheet to water repellent finishing, it is possible to provide the absorbent pad for men having a small number of parts with simple configuration.

The twenty-third alternative is the absorbent pad for men according to the eighteenth or nineteenth alternatives, wherein the second gather sheet extends from a direction of the other end out of the front and rear ends of the top sheet toward the central direction of the top sheet.

According to the twenty-third alternative, since the second gather sheet extends from a direction of the other end out of the front and rear ends of the top sheet toward the central direction of the top sheet, it is possible to ensure fixing the second gather sheet to the other end of the top sheet. The absorbent pad for men that can prevent leakage of urine can be provided, accordingly.

The twenty-fourth alternative is the absorbent pad for men according to the twenty-third alternative, wherein a liquid passage portion is provided by providing a gap between the top sheet and the second gather sheet in a portion in which the absorbent pad for men is folded in the longitudinal direction.

According to the twenty-fourth alternative, a liquid passage portion is provided by providing a gap between the top sheet and the second gather sheet in a portion in which the absorbent pad for men is folded in the longitudinal direction. Therefore, the second gather sheet is bonded to the top sheet in the bonded portions, the urine can be transmitted by the liquid permeable second gather sheet and smoothly absorbed by the absorbent member on the folded side through the liquid passage portion provided between the top sheet ad the second gather sheet. It is, therefore, possible to provide the absorbent pad for men that can prevent leakage of urine.

The twenty-fifth alternative is the absorbent pad for men according to any one of the seventeenth to twenty-fourth alternatives, wherein the back sheet is liquid impermeable.

According to the twenty-fifth alternative, since the back sheet is liquid impermeable, the urine absorbed by the absorbent member does not leak outside and contaminate an underwear or the like.

The twenty-six alternative is the absorbent pad for men according to any one of the seventeenth to twenty-fourth alternatives, wherein the back sheet is liquid permeable.

According to the twenty-six alternative, the back sheet is liquid permeable. Due to this, if the absorbent pad for men is used together with an unfolded paper diaper, an absorbent pad of the unfolded paper diaper can be also used and an absorption amount of the urine can be increased. If the absorbent pad for men is used at midnight, it can be used until the next morning without need to replace the pad with a new one.

### BRIEF DESCRIPTION OF THE DRAWINGS

Fig. 1 is a plan view of an absorbent pad for men according to a first embodiment.
Fig. 2 is a sectional view of the absorbent pad for men shown in Fig. 1 taken along a line X-X' of Fig. 1.
Fig. 3 is an explanatory view for a manner of inserting male genitals into the absorbent pad for men, i.e., Fig. 3(A) is a schematic sectional view for explaining a state of inserting the male genitals thereinto with a wearer lying on his back, and Fig. 3(B) is a schematic sectional view for explaining a state of inserting the upward male genitals thereinto with the wearer lying on his back.
Fig. 4 is a plan view of an unfolded paper diaper.
Fig. 5 is a sectional view of an absorbent pad for men as another example of the first embodiment.
Figs. 6(A) and 6(B) are plan views of an absorbent pad for men as yet another example of the first embodiment.
Fig. 7 is a plan view of an absorbent pad for men according to an alternative which is not part of the present invention.
Fig. 8 is a sectional view of the absorbent pad for men shown in Fig. 7 taken along a line X-Y of Fig. 7.
Fig. 9 is a schematic sectional view for explaining a manner of inserting male genitals into the absorbent pad for men shown in Fig. 7.
Fig. 10 is a sectional view of another absorbent pad for men according to the second embodiment.
Fig. 11 is a schematic sectional view for explaining a manner of inserting male genitals into the absorbent pad for men shown in Fig. 10.
Fig. 12 is a sectional view of yet another absorbent pad for men according to the second embodiment.
Fig. 13 is a schematic sectional view for explaining a manner of inserting male genitals into the absorbent pad for men shown in Fig. 12.
Fig. 14 is a sectional view that depicts a modification of the absorbent pad for men shown in Fig. 12.
Fig. 15 is a plan view of an absorbent pad for men according to an alternative which is not part of the present invention.
Fig. 16 is a sectional view of the absorbent pad for men shown in Fig. 15 taken along a line X1-X2 of Fig. 15.
Fig. 17 is a sectional view of the absorbent pad for men shown in Fig. 15 taken along a line Y1-Y2 of Fig. 15.
Fig. 18 is a sectional view of the absorbent pad for men shown in Fig. 15 taken along a line Z1-Z2 of Fig. 15.
Fig. 19 is a schematic sectional view for explaining a manner of inserting male genitals into the absorbent pad for men shown in Fig. 15.
Fig. 20 is a sectional view that depicts an absorbent pad for men as another example of an alternative which is not part of the invention.
Fig. 21 is a sectional view that depicts an absorbent pad for men as yet another example of the third embodiment.
Fig. 22 is a sectional view that depicts an absorbent pad for men as a modification of the pad shown in Fig. 20.
Fig. 23 is a sectional view of an absorbent pad for men as another example of the third embodiment in a width direction.
Fig. 24 is a sectional view of the absorbent pad for men shown in Fig. 22 in a longitudinal direction.
Fig. 25 is a schematic plan view that depicts an example of folding back coupled portions of sides of the absorbent pad for men shown in Fig. 15 toward an opening side.
Figs. 26(A) and 26(B) are a perspective view and a sectional view of a conventional absorbent pad for men, respectively.
Fig. 27(A) and 27(B) are a perspective view and a schematic sectional view of another conventional absorbent pad for men, respectively.

### BEST EMBODIMENT FOR CARRYING OUT THE INVENTION

### < EMBODIMENT ACCORDING TO THE INVENTION>

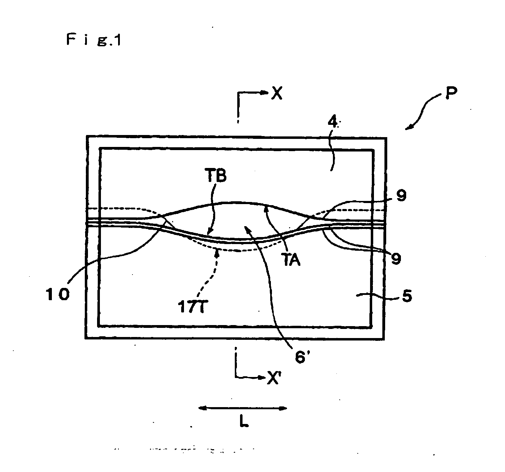

An embodiment of the present invention will be described hereinafter with reference to Figs. 1 to 6.

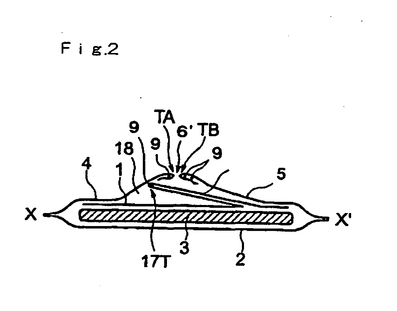

Fig. 1 is a plan view of an absorbent pad for men as one example of the invention, and Fig. 2 is a sectional view of the absorbent pad for men taken along a line X-X' of Fig. 1. An absorbent pad for men P includes a rectangular liquid permeable top sheet 1, a liquid impermeable back sheet 2, an absorbent member 3 arranged between the top sheet 1 and the back sheet 2, and a pair of liquid impermeable gather sheets arranged on the top sheet 1 and facing each other, i.e., a first gather sheet (corresponding to the other gather sheet according to claims 1 to 10) 4 and a second gather sheet (corresponding to one gather sheet according to claims 1 to 10) 5. Tip ends TA and TB of the respective gather sheets 4 and 5 are formed to face each other along a longitudinal direction L of the absorbent pad for men P.

One elastic member 9 consisting of thread rubber is bonded to the tip end TA of the first gather sheet 4 in the longitudinal direction L in an expanded state. Two elastic members 9 consisting of the thread rubber are bonded to the tip end TB of the second gather sheet 5 in the longitudinal direction L in expanded states.

In this embodiment, an auxiliary gather sheet (corresponding to an auxiliary sheet according to claims 1 to 10) 10 for diffusing liquefied excrement is provided integrally with the top sheet 1 between the top sheet 1 and the second gather sheet 5. More specifically, the liquid permeable auxiliary gather sheet 10 extends from one gather sheet 5 side to the other gather sheet 4 side between the top sheet 1 and the gather sheets 4 and 5. A genital holding space 18 is formed in a space surrounded by the first gather sheet 4, the auxiliary gather sheet 10, and the top sheet 1. An opening 6' for inserting the male genitals into the genital holding space 18 is formed by the tip end TA of the first gather sheet 4 and a tip end 17T of the auxiliary gather sheet 10. This opening 6' is formed to be unevenly distributed to the first gather sheet 4 side.

The tip end 17T of the auxiliary gather sheet 10 is provided to enter below the first gather sheet 4. This auxiliary gather sheet 10 is formed by angle-folding the top sheet 1. Namely, the top sheet 1 and the auxiliary gather sheet 10 are equal in material. The elastic member 9 is fixedly provided on the tip end 17T of the auxiliary gather sheet 10 in an expanded state along a ridgeline of the angle-folded sheet 1. The tip end 17T of the auxiliary gather sheet 10 may be generally at the same position as either the tip end TB of the gather sheet 5 or the tip end TA of the gather sheet 4.

The number of elastic members 9 provided on the tip end 17T of the auxiliary gather sheet 10 may be one or two or more. If a plurality of elastic members 9 are provided, it is possible to further reduce a gap between the male genitals and the absorbent pad for men P and thereby provide the absorbent pad for men capable of further preventing leakage of urine.

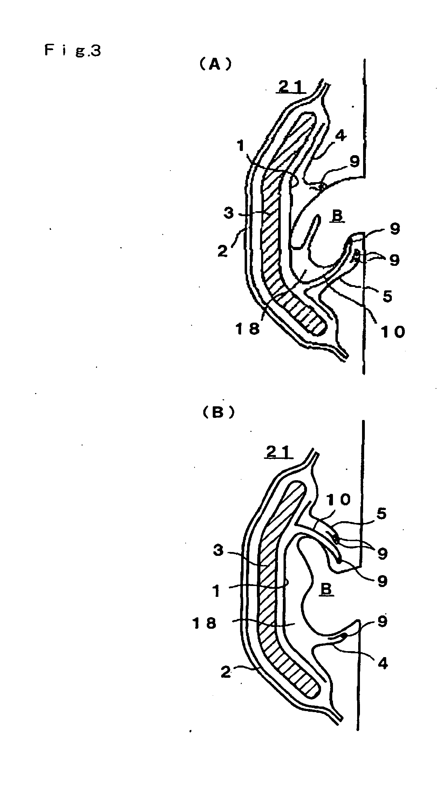

If the absorbent pad for men P thus configured is used, the downward male genitals B are inserted into the absorbent pad for men P from the opening 6' with the wearer lying on his back, put on the auxiliary gather sheet 10, and held by the genital holding pace 18 as shown in Fig. 3(A).

If so, the auxiliary gather sheet 10 including the elastic members 9 and the gather sheet 4 including the elastic member 9 enable the male genitals B to be securely held in the absorbent pad for men P.

At this moment, the male genitals are put on the auxiliary gather sheet 10. Due to this, even if the wearer urinates while lying on his back, the liquid permeable auxiliary gather sheet 10 abates the force of the urine white permeating the urine to thereby diffuse the urine throughout the auxiliary gather sheet 10. In addition, the gather sheet 5 including the two auxiliary elastic members 9 outside the auxiliary gather sheet 10 securely receives the urine, so that leakage of the urine from the opening 6' does not occur.

Alternatively, the upward male genitals B may be inserted into the absorbent pad for men P from the opening 6', and held by the genital holding space 18 so that the penis is put on the auxiliary gather sheet 10. In this case, as shown in Fig. 3(B), the absorbent pad for men P is used by turning the pad P by 180 degrees with respect to the state shown in Fig. 3(A). Namely, the upward male genitals B are inserted into the absorbent pad for men P from the opening 6' while the wearer lies on his back, put on the auxiliary gather sheet 10, and held by the genital holding space 18. If so, similarly to Fig. 3(A), the male genitals B are securely wrapped in the auxiliary gather sheet 10 and the gather sheets 4 and 5 including the elastic members 9.

In this case, the male genitals B are put on the auxiliary gather sheet 10. Due to this, even if the wearer urinates while lying on his back, the liquid permeable auxiliary gather sheet 10 abates the force of the urine while permeating the urine to thereby diffuse the urine throughout the auxiliary gather sheet 10. Leakage of the urine from the opening 6' does not, therefore, occur.

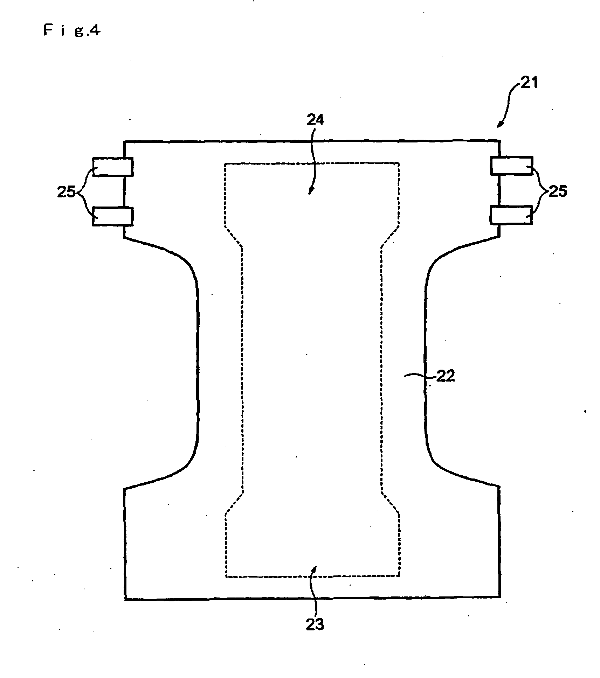

Thereafter, as shown in Fig. 4, if necessary, the wearer's hip is put on a top sheet 22 of a back body 24 of an unfolded paper diaper 21, a front body 23 thereof is folded and abutted on the wearer's abdomen who wears the absorbent pad for men P, and stopper members 25 such as both-sided tapes or mechanical fasteners are fixed onto a back sheet of the front body 23 of the unfolded paper diaper 21, whereby the wearer wears the unfolded paper diaper 21.

The absorbent pad for men P in this embodiment is configured as stated above. Due to this, even if the wearer urinates while lying on his back, the auxiliary gather sheet 10 abates the force of the urine and promptly diffuses the urine. The urine, therefore, does not leak from the opening 6'. The absorbent pad for men P in this embodiment can, therefore, prevent the liquefied excrement such as the urine from leaking outside from the opening 6', irrespective of the direction of the male genitals B.

In addition, since the male genitals B can be held in a free state, it is possible to make the wearer less feel something strange. Besides, since the absorbent pad for men P according to the present invention enables the male genitals B to be held by the genital holding space 18, the pad P can follow a wearer's motion.

Further, in this embodiment, the absorbent pad for men P is preferably folded into two at a center in the longitudinal direction L and wrapped up. If so, when the absorbent pad P is opened and attached to the unfolded paper diaper or the like, it is advantageously possible to easily determine the center of the pad P and easily abut the center of the pad P against the male genitals. Besides, the liquefied excrement can be easily introduced toward the center of the absorbent member through a valley generated by a folded track of the absorbent pad P folded into two.

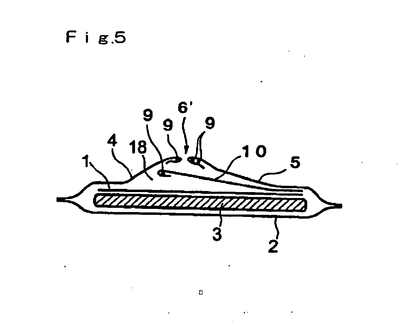

In this embodiment, the auxiliary gather sheet 10 is provided integrally with the top sheet 1. However, the present invention is not limited to this embodiment. As shown in Fig. 5, the auxiliary gather sheet 10 may be provided separately from the top sheet 1.

In this embodiment, the instance of attaching the unfolded paper diaper to the wearer who wears the absorbent pad for men P has been described. However, the present invention is not limited to this embodiment. The absorbent pad for men P may be used together with a briefs type paper diaper, a briefs type underwear or the like. Accordingly, the back sheet 2 of the absorbent pad for men P preferably includes two-sided adhesive tapes or male parts of mechanical fasteners. If so, it is advantageously possible to prevent the absorbent pad for men P from being shifted because of the wearer's motion.

In this embodiment, the back sheet 2 is liquid impermeable. However, the present invention is not limited to this embodiment. The back sheet 2 may be liquid impermeable.

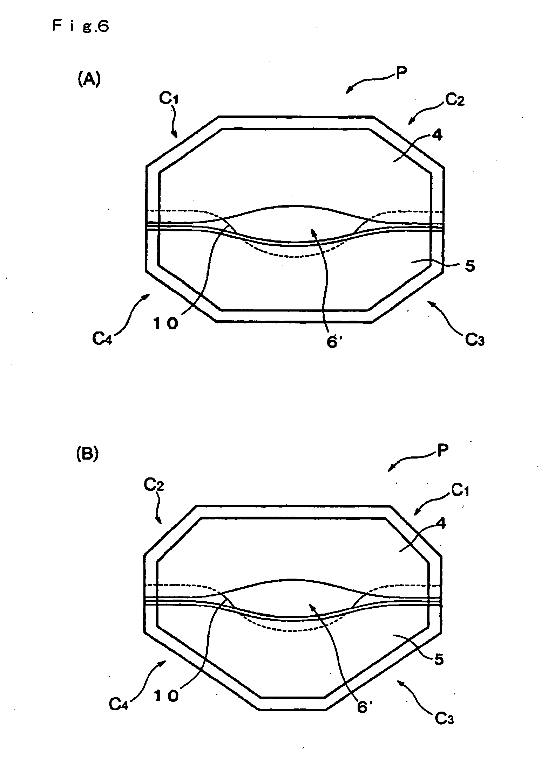

In this embodiment, the absorbent pad for men P is formed into a rectangle. However, the present invention is not limited to this embodiment. As shown in Fig. 6(A), the absorbent pad for men P may be formed into a rectangle having corners C1 to C4 uniformly notched. Alternatively, as shown in Fig. 6(B), the two corners C3 and C4 on both ends of one major side may be notched larger than the two corners C1 and C2 on both ends of the other major side, or only the corners C3 and C4 may be notched. If so, when the wearer wears the absorbent pad for men P, the notched parts can improve the wearer's sense that the hip joint is fit to the pad P. Needless to say, the same advantages can be attained even if only corners of the rectangular absorbent member 3 are notched without notching those of the top sheet 1 and the back sheet 2.

### < ALTERNATIVES OF ABSORBENT PADS NOT COVERED BY THE PRESENT INVENTION>

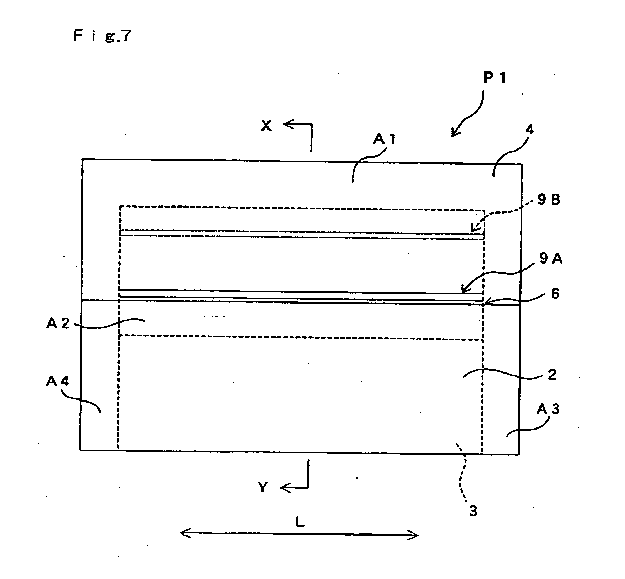

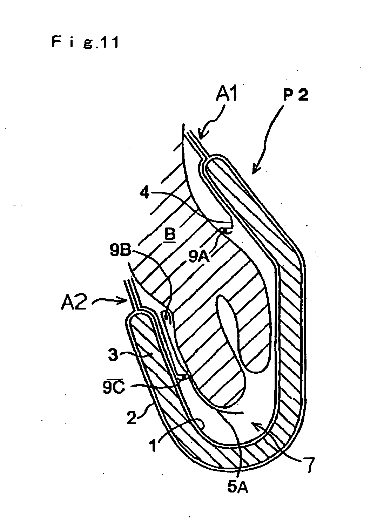

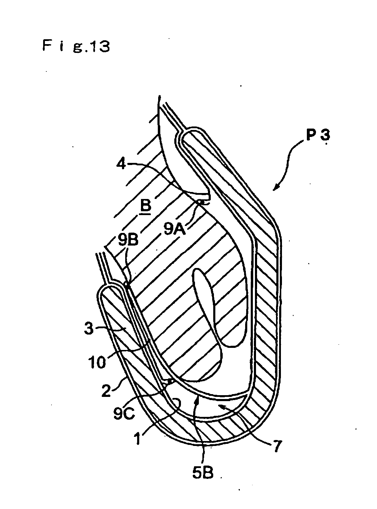

An alternative of an absorbent pad will be described with reference to Figs. 7 to 14.

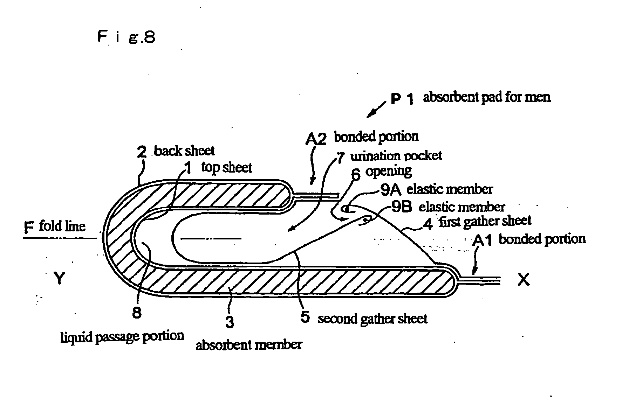

Fig. 7 is a plan view of an absorbent pad for men t, and Fig. 8 is a sectional view of the absorbent pad for men taken along a line X-Y of Fig. 7. An absorbent pad for men P1 includes a rectangular liquid permeable top sheet 1, a liquid impermeable back sheet 2 equal in shape to the top sheet 1, an absorbent member 3 arranged between the top sheet 1 and the back sheet 2. Four sides of the top sheet 1 and the back sheet 2 are bonded to one another by hot melt adhesive or the like, and bonded portions are denoted by A1 to A4, respectively. One end of a liquid impermeable first gather sheet 4 is bonded to the bonded portion A1, and one end of a liquid impermeable second gather sheet 5 is bonded to the bonded portion A2.

The first gather sheet 4 and the second gather sheet 5 face each other, thereby forming an opening 6 for inserting male genitals. This opening 6 is formed to be unevenly distributed to the first gather sheet 4 side of the absorbent pad for men P1. If this absorbent pad P1 is folded back on a fold line F to bond the bonded portions A3 and A4 along a direction of tip ends of the first gather sheet 4 and the second gather sheet 5, with the top sheet located inside, a urination pocket 7 is formed. A gap is formed between the top sheet 1 and the second gather sheet 5 in a folded portion within the urination pocket 7, thereby forming a liquid passage portion 8. Elastic members 9A and 9B consisting of thread rubber are bonded to the tip ends of the first gather sheet 1 and the second gather sheet 5 along the longitudinal direction L in expanded states, respectively.

In this alternative, the first gather sheet 4 and the second gather sheet 5 are provided on the top sheet 1 so that liquefied excrement does not leak outside. An end of the first gather sheet 4 on which side the elastic member 9A is not provided is bonded to the top sheet 1 in the bonded portion A1, and an end of the second gather sheet 5 on which side the elastic member 9B is not provided is bonded to the back sheet 2 in the bonded portion A2.

In this alternative, the tip end of the first gather sheet 4 is configured to be overlapped with that of the second gather sheet 5. The opening 6 is provided to insert the male genitals. In this alternative, the opening 6 is formed to be unevenly distributed to the first gather sheet 4 side of the absorbent pad P1.

Further, in this alternative, the end of the first gather sheet 4 on which side the elastic member 9A is not provided is bonded to the top sheet 1 in the bonded portion A1, and the end of the second gather sheet 5 on which side the elastic member 9B is not provided is bonded to the back sheet 2 in the bonded portion A2. However, the bonded portions in which the first gather sheet 4 and the second gather sheet 5 are bonded to the top sheet 1 and the back sheet 2, respectively, are not limited to the bonded portions A1 and A2.

The numbers of the elastic members 9A and 9B provided on the tip ends of the first gather sheet 4 and the second gather sheet 5, respectively, may be one or two or more. If a plurality of each of elastic members 9A and 9B are provided, it is possible to more strongly hold down the male genitals, eliminate the gap between the male genitals and the absorbent pad for men P1, and provide the absorbent pad for men which can further prevent leakage of urine.

If the absorbent pad for men P1 thus configured is used, the downward male genitals B are inserted from the opening 6 of the absorbent pad for men P1 with the wearer standing, stored so that the second gather sheet 5 is between the scrota and the top sheet 1 on the side on which the pad P1 is not folded, and held by the pad P1.

At this time, the first gather sheet 4 is fit to an upper portion of a surface of the penis by the elastic member 9A and the second gather sheet 5 is fit to a rear surface of the scrota by the elastic member 9B, thus preventing leakage of urine.

If the wearer urinates and excretes a large amount of urine at one time, an amount of the urine corresponding to a speed exceeding an absorption speed of the absorbent member 3 is temporarily received in the urination pocket 7. Since the second gather sheet 5 is bonded to the top sheet 1 in the bonded portion A2, the urine received in the urination pocket 7 can be smoothly absorbed by the absorbent member 3 on the folded side through the liquid passage portion 8 provided between the top sheet 1 and the second gather sheet 5. In addition, the liquid impermeable second gather sheet 5 provided to extend from the end of the absorbent member 3 on the folded side can prevent the urine from leaking from within the urination pocket 7.

The male genitals B are inserted between the second gather sheet 5 and the top sheet 1. Due to this, even if the wearer urinates while lying on his back, the urine staying in the liquid passage portion 8 is sequentially absorbed and held by the absorbent member 3. At this moment, the elastic member 9B enables the second gather sheet 5 to be fit to the rear surface of the scrota, so that the urine does not leak outside.

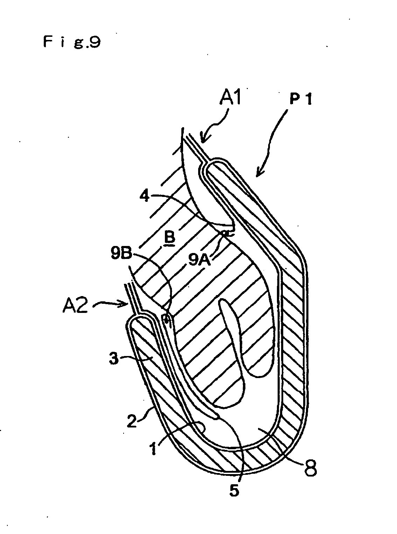

Alternatively, the upward male genitals B may be inserted between the second gather sheet 5 and the top sheet 1 from the opening 6. In this case, the absorbent pad for men P1 is used with its direction vertically changed by 180 degrees from that shown in Figs. 3(A) and 3(B). Namely, the upward male genitals B are inserted from the opening 6 of the absorbent pad for men P1 with the wearer lying on his back and inserted and held between the second gather sheet 5 and the top sheet 1. If so, similarly to Fig. 9, the male genitals are securely wrapped up between the first gather sheet 4 including the elastic member 9A and the second gather sheet 5 including the elastic member 9A.

In this case, similarly to the above, the male genitals B are inserted and held between the second gather sheet 5 and the top sheet 1. Due to this, even if a large amount of urine is temporarily excreted during urination, the excreted urine is prevented from leaking outside by the first gather sheet 4 and the second gather sheet 5 and absorbed and held by the absorbent member 3.

Thereafter, if necessary, the wearer's hip may be put on a top sheet of a back body of an unfolded paper diaper, a front body thereof may be folded and abutted on the wearer's abdomen who wears the absorbent pad for men P1, and stopper members such as both-sided tapes or mechanical fasteners may be fixed onto a back sheet of the front body of the unfolded paper diaper, whereby the wearer may wear the unfolded paper diaper 21.

In this case, a liquid permeable sheet may be used as the back sheet 2 of the absorbent pad for men P1 to make an absorbent member of the unfolded paper diaper absorb the urine so as to lengthen utilization time.

The absorbent pad for men P1 may be used together with a briefs type paper diaper, a briefs type underwear or the like in place of the unfolded paper diaper. Accordingly, the back sheet 2 of the absorbent pad for men P1 preferably includes two-sided adhesive tapes or male parts of mechanical fasteners. If so, it is advantageously possible to prevent the absorbent pad for men P1 from being shifted because of the wearer's motion.

Since the absorbent pad for men according to this embodiment can hold the male genitals B in a free state, it is possible to make the wearer less feel something strange. Besides, since the absorbent pad for men P1 according to the present invention enables the male genitals B to be securely held by the first gather sheet 4 including the elastic member 9A and the second gather sheet 5 including the elastic member 9B, the pad P1 can follow the wearer's motion.

Further, the absorbent pad for men P1 is preferably folded into two at a center in the longitudinal direction L and wrapped up. If so, when the absorbent pad P1 is opened and attached to the unfolded paper diaper or the like, it is advantageously possible to easily determine the center of the pad P1 and easily abut the center of the pad P1 against the male genitals B. Besides, the liquefied excrement can be easily introduced toward the center of the absorbent member 3 through a valley generated by a folded track of the absorbent pad P1 folded into two.

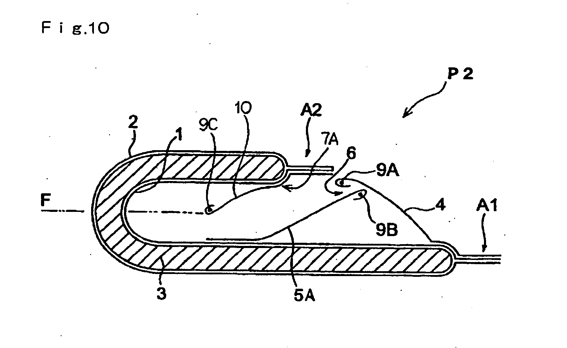

Another example of the absorbent pad for men according to this second embodiment will next be described with reference to Fig. 10. The top sheet 1, the back sheet 2, the absorbent member 3, and the first gather sheet 4 are equal in shape, material, and configuration to those stated above. In this example, a liquid impermeable second gather sheet 5A is not formed to extend up to the bonded portion A2 along the top sheet 1. Besides, a liquid impermeable auxiliary gather sheet 10 for preventing leakage of the urine staying in the urination pocket 7 is provided on the top sheet 1 on the absorbent pad folded side near an opening 7A of the urination pocket 7.

An elastic member 9C such as thread rubber is provided on a tip end of this auxiliary gather sheet and a material for the auxiliary gather sheet 10 is the same as that for the first gather sheet 4 and the second gather sheet 5 according to the second embodiment. The second gather sheet 5A in this example has both sides bonded to the top sheet 1 in the bonded portions A3 and A4, whereby the second gather sheet 5A is formed integrally with an absorbent pad for men P2. The first gather sheet 4 and the second gather sheet 5A form the opening 6 for inserting the male genitals B. If the absorbent pad for men P2 is folded back on the fold line F to bond the bonded portions A3 and A4, with the top sheet 1 located inside, the urination pocket 7 is formed.

If the absorbent pad for men P2 thus configured is used, the downward male genitals B are inserted from the opening 6 of the absorbent pad for men P2 with the wearer standing, inserted between the second gather sheet 5A and the top sheet 1, and held by the absorbent pad for men P2 with the second gather sheet 5A abutted on the rear surface of the scrota.

At this time, the first gather sheet 4 is fit to an upper portion of the surface of the penis by the elastic member 9A and the second gather sheet 5A is fit to the rear surface of the scrota by the elastic member 9B, whereby the entire male genitals B are securely held by the absorbent pad for men P2. Besides, the elastic member 9C enables the auxiliary gather sheet 10 to be fit to the rear surface of the scrota. If the wearer urinates and excretes a large amount of urine at one time, the urine excreted from the male genitals at a breath is received in the urination pocket 7, and can be smoothly absorbed by the absorbent member on the folded side through a gap formed between the top sheet and the second gather sheet. In addition, the liquid impermeable auxiliary gather sheet provided to extend from above the top sheet 1 on the end of the absorbent member on the folded side can prevent the urine from leaking from within the urination pocket.

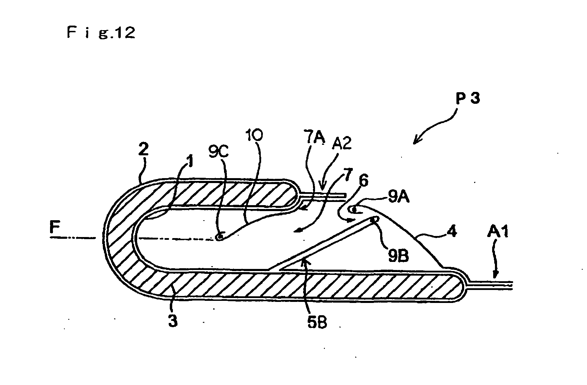

Yet another example of the absorbent pad for men according to this alternative will be described with reference to Fig. 12. The top sheet 1, the back sheet 2, the absorbent member 3, the first gather sheet 4, and the auxiliary gather sheet 10 are equal in shape, material, and configuration to those stated above. In this example, a second gather sheet 5B extends toward the top sheet 1, thereby forming the second gather sheet 5B integrally with the top sheet 1. The elastic member 9B is provided on a tip end of this second gather sheet 5B, and the first gather sheet 4 and the second gather sheet 5B form the opening 6 for inserting the male genitals B.

An absorbent pad for men P3 is folded back on the fold line F to bond the bonded portions A3 and A4, with the top sheet 1 located inside, whereby the urination pocket 7 is formed.

If the absorbent pad for men P3 thus configured is used, the downward male genitals B are inserted from the opening 6 of the absorbent pad for men P3 with the wearer standing, inserted between the second gather sheet 5B and the top sheet 1, and held by the absorbent pad for men P3 with the second gather sheet 5B abutted on the rear surface of the scrota.

At this time, the first gather sheet 4 is fit to the upper portion of the surface of the penis by the elastic member 9A and the second gather sheet 5B is fit to the rear surface of the scrota by the elastic member 9B, whereby the male genitals B are entirely and securely held by the absorbent pad for men P3. Besides, the elastic member 9C enables the auxiliary gather sheet 10 to be fit to the rear surface of the scrota. If the wearer urinates and excretes a large amount of urine at one time, then the force of the urine is abated by the liquid permeable second gather sheet 5B and absorbed by the entire absorbent member 3 while being permeated to the top sheet 1 on both sides through the second gather sheet 5B. If the urine leaks from the second gather sheet 5B, the urine can temporarily stay on a tip end of the urination pocket 7.

Since the auxiliary gather sheet 10 is bonded to the top sheet 1 in the pocket opening 7A, the urine staying on the tip end of the urination pocket 7 can be held without leaking outside. The urine temporarily staying on the tip end of the urination pocket 7 is sequentially and uniformly absorbed and held by the entire absorbent member 3 through the top sheet 1.

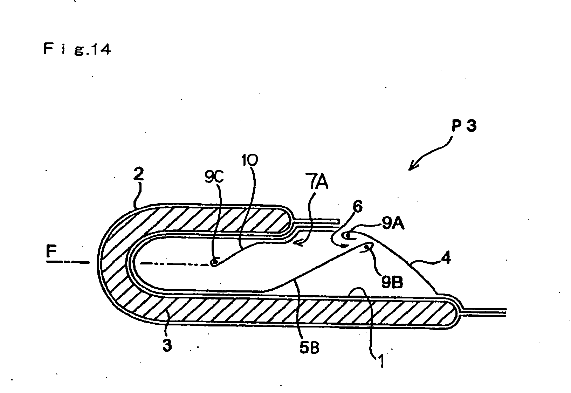

In this example, the second gather sheet 5B is provided to extend from the top sheet 1 integrally with the top sheet 1. Alternatively, the second gather sheet 5B may be provided separately from the top sheet as shown in Fig. 14.

In the three examples described above, the back sheet 2 is liquid impermeable. However, the back sheet 2 may be liquid permeable.

Further, the tip end of the first gather sheet 4 is overlapped with that of the second gather sheet 5. Alternatively, the tip ends of the first gather sheet 4 and the second gather sheet may be located generally at the same position.

The absorbent pad for men P is formed into a rectangle. However, the absorbent pad for men P may be formed into a rectangle having corners uniformly notched. If so, when the wearer wears one of the absorbent pad for mens P1 to P3, the notched parts can improve the wearer's sense that the hip joint is fit to the pad P. Needless to say, the same advantages can be attained even if only corners of the rectangular absorbent member 3 are notched without notching those of the top sheet 1 and the back sheet 2.

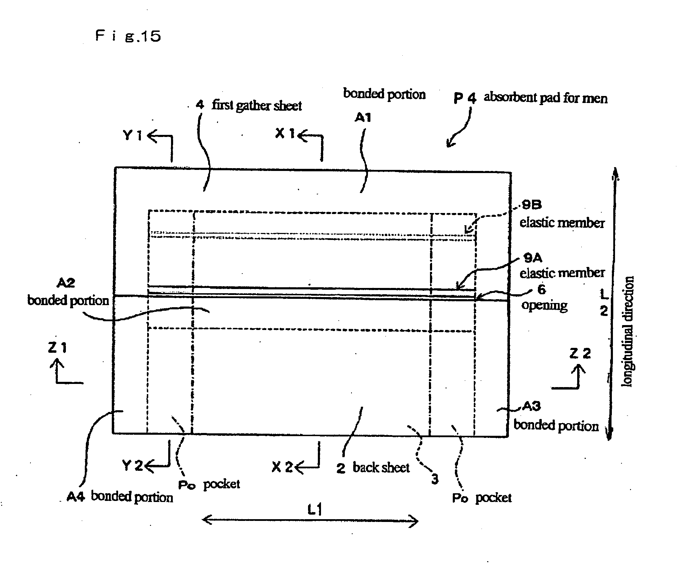

Referring to Fig. 15 to 25, a further alternative of an absorbent pad will be described.

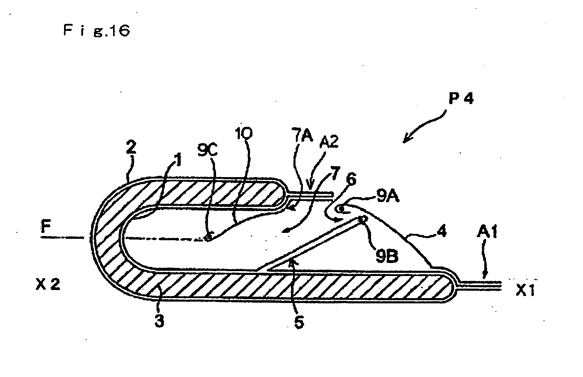

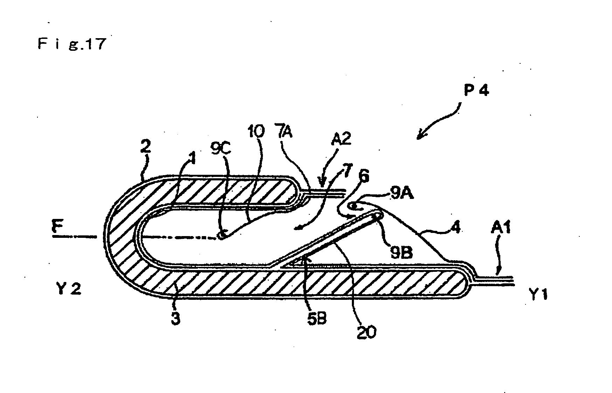

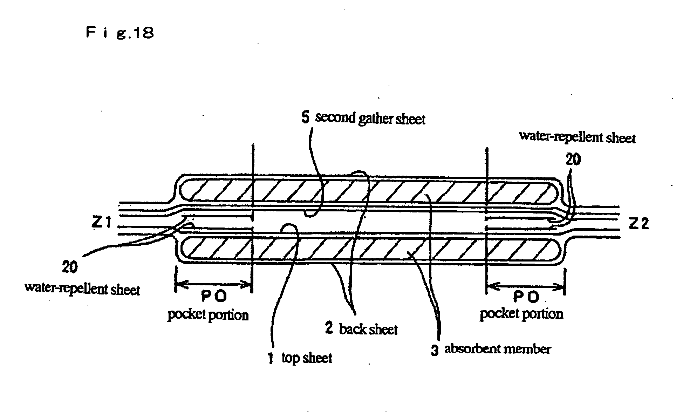

Fig. 15 is a plan view of an absorbent pad for men. Fig. 16 is a sectional view of the absorbent pad for men taken along a line X1-X2 of Fig. 15. Fig. 17 is a sectional view of the absorbent pad for men taken along a line Y1-Y2 of Fig. 15. Fig. 18 is a sectional view of the absorbent pad for men taken along a line Z1-Z2 of Fig. 15. An absorbent pad for men P4 is configured so that a rectangular liquid impermeable back sheet 2, a rectangular absorbent member 3, and a rectangular liquid permeable top sheet 1 are superimposed from below in this order, and so that peripheral edges A1 to A4 of the back sheet 2 and the top sheet 1 are bonded to one another by hot melt adhesive or the like. The absorbent member 3 is smaller than the back sheet 2 by as much as the bonded portions A1 to A4. The top sheet 1 is smaller than the back sheet 2. By so configuring, the back sheet 2 can prevent permeation of urine from a pocket PO to be described later.

The liquid impermeable first gather sheet 4 is provided from one end of front and rear end of the top sheet 1 in a longitudinal direction L2, i.e., from the bonded portion A1 toward a central portion of the top sheet 1 in the longitudinal direction L2. The second gather sheet 5 is formed by angle-folding the top sheet 1 from the other end direction, i.e., a direction of the bonded portion A2 toward the central portion of the top sheet 1 in the longitudinal direction L2. The thread rubber 9B that is an elastic member is bonded to an interior of a folded portion in an expanded state. The opening 6 is formed so that tip ends of the first gather sheet 4 and the second gather sheet 5 are overlapped with each other.

As shown in Figs. 17 and 18, a water-repellent sheet (water-repellent strip) 20 is provided on each absorbent member side edge VE in the longitudinal direction L2 of the top sheet 1. The absorbent pad for men P4 is folded into two on the fold line F, the bonded portions A1, A3, and A4 are further bonded and overlapped with one another by the hot melt adhesive, thereby forming the pocket PO in each absorbent member side edge VE. In addition, the liquid impermeable auxiliary gather sheet 10 for preventing leakage of urine staying in the urination pocket 7 is provided near the bonded portion A2 on the folded side of the absorbent pad.

The absorbent pad for men P4 is folded into two in the longitudinal direction L2, and the bonded portions A1 to A4 are further bonded to the first gather sheet 4 and the second gather sheet 5, thereby forming the pockets PO in both sides VE of the absorbent member, respectively.

The elastic member 9C such as thread rubber is provided on the tip end of this auxiliary gather sheet 10, and a material for this auxiliary gather sheet 10 is the same as that for the first gather sheet and the second gather sheet 5 stated above. The both sides of the second gather sheet 5 in this example are bonded to the absorbent pad for men P4 in the bonded portions A3 and A4, thereby forming the second gather sheet 5 integrally with the absorbent pad for men P4.

Alternatively, the top sheet on the both sides of the absorbent (pocket PO formation portions) VEs may be subjected to water repellent finish instead of forming the water-repellent sheets 20.

In the examples shown in Figs. 15 to 18, the opening 6 is formed to be unevenly distributed to the first gather sheet 4 of the absorbent pad for men P4. Alternatively, the opening 6 may be formed at a center of the pad P4 in the longitudinal direction L2. The elastic members 9A and 9B of the thread rubber are bonded to tip ends of the first gather sheet 4 and the second gather sheet 5 along a width direction L1 in expanded states, respectively.

The numbers of the elastic members 9A and 9B provided on the tip ends of the first gather sheet 4 and the second gather sheet 5, respectively, may be one or two or more. If a plurality of each of elastic members 9A and 9B are provided, it is possible to more strongly hold down the male genitals, eliminate the gap between the male genitals and the absorbent pad for men P4, and provide the absorbent pad for men which can further prevent leakage of urine.

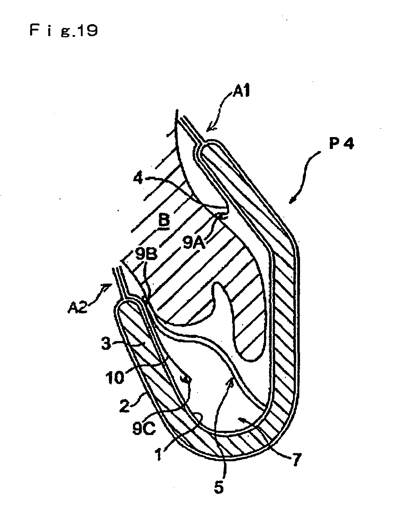

If the absorbent pad for men P4 thus configured is used, the downward male genitals B are inserted from the opening 6 of the absorbent pad for men P4 with the wearer standing, stored so that the second gather sheet 5 is between the scrota and the top sheet 1 on the side on which the pad P4 is not folded, and held by the pad P4, as shown in Fig. 19, for example.

At this time, the first gather sheet 4 is fit to the upper portion of the surface of the penis by the elastic member 9A and the second gather sheet 5 is fit to the rear surface of the scrota by the elastic member 9B, whereby the entire male genitals B are securely held by the absorbent pad for men P4. Besides, the elastic member 9C enables the auxiliary gather sheet 10 to be fit to the rear surface of the scrota.

If the wearer urinates and excretes a large amount of urine at one time, the urine exerted from the male genitals at a breath is received in the urination pocket 7 through the second gather sheet 5, temporarily stays in the urination pocket 7 provided between the top sheet 1 and the second gather sheet 5, and can be smoothly absorbed by the absorbent member 3. In addition, the liquid impermeable auxiliary gather sheet 10 provided on the top sheet in the bonded portion A2 on the folded side can prevent the urine from leaking from within the urination pocket 7.

Furthermore, the pockets PO are formed in the absorbent member side edges VEs of the absorbent pad for men P4 in this embodiment, and the water-repellent sheets 20 are bonded on the top sheet therein. Due to this, even if the wearer urinates while lying down, the urinate that is not absorbed by the absorbent member 3 temporarily stays in the pockets PO and do not leak outside.

Alternatively, the upward male genitals B may be inserted between the second gather sheet 5 and the top sheet 1 from the opening 6 of the absorbent pad for men P4. In this case, the absorbent pad for men P4 is used with its direction, vertically changed by 180 degrees from that shown in Fig. 19. Namely, the upward male genitals B are inserted from the opening 6 of the absorbent pad for men P4 with the wearer lying on his back and inserted and held between the second gather sheet 5 and the top sheet 1. If so, similarly to Fig. 19, the male genitals B are securely wrapped up between the first gather sheet 4 including the elastic member 9A and the second gather sheet 5 including the elastic member 9A.

In this case, similarly to the above, the male genitals B are inserted and held between the second gather sheet 5 and the top sheet 1. Due to this, even if a large amount of urine is temporarily excreted during urination, the excreted urine is prevented from leaking outside by the first gather sheet 4 and the second gather sheet 5 and absorbed and held by the absorbent member 3.

In this example, the back sheet 2 is liquid impermeable. However, the back sheet 2 may be liquid permeable. In this case, the water-repellent sheets 20 are provided to extend toward the bonded portions 3 and A4 so as to prevent lateral leakage of urine when the wearer lies down.

If the absorbent pad for men P4 having the liquid permeable back sheet 2 is used, then the wearer's hip may be put on a top sheet of a back body of an unfolded paper diaper, a front body thereof may be folded and abutted on the wearer's abdomen who wears the absorbent pad for men P4, and stopper members such as both-sided tapes or mechanical fasteners may be fixed onto a back sheet of the front body of the unfolded paper diaper, whereby the wearer may wear the unfolded paper diaper. If so, the urinate can be also absorbed by the absorbent member of the unfolded paper diaper, and wearing time can be lengthened. Therefore, caretaker's labor for replacing the paper diaper with a new one at midnight can be saved.

The absorbent pad for men P may be used together with a briefs type paper diaper, a briefs type underwear or the like in place of the unfolded paper diaper. Accordingly, the back sheet 2 of the absorbent pad for men P preferably includes two-sided adhesive tapes or male parts of mechanical fasteners. If so, it is advantageously possible to prevent the absorbent pad for men P1 from being shifted because of the wearer's motion.

Since the absorbent pad for men can hold the male genitals B in a free state, it is possible to make the wearer less feel something strange. Besides, since the absorbent pad for men P according to the present invention enables the male genitals B to be securely held by the first gather sheet 4 including the elastic member 9A and the second gather sheet 5 including the elastic member 9B, the pad P can follow the wearer's motion.

Further, the absorbent pad for men P is preferably folded into two at a center in the longitudinal direction L1 shown in Fig. 15 and wrapped up. If so, when the absorbent pad P is opened and attached to the unfolded paper diaper or the like, it is advantageously possible to easily determine the center of the pad P and easily abut the center of the pad P against the male genitals B. Besides, the liquefied excrement can be easily introduced toward the center of the absorbent member 3 through a valley generated by a folded track of the absorbent pad P folded into two.

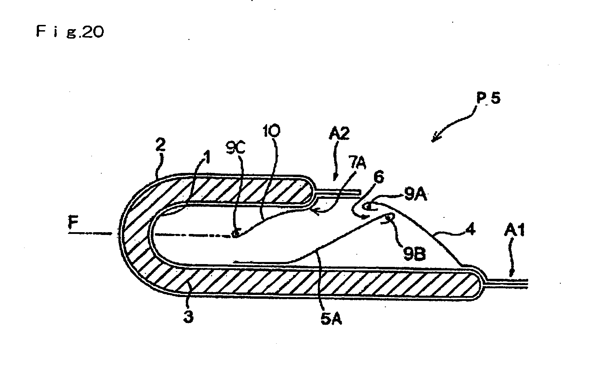

Another example of the absorbent pad for men will next be described with reference to Fig. 20. An absorbent pad for men P5 in this example differs from that in the preceding example in shape and attachment of the second gather sheet 5A. Since the remaining configurations of the pad P5 are the same as those in the preceding example, they will not be described herein. The absorbent pad for men P5 in this example is configured so that the second gather sheet 5A is formed to be fixed near the central portion of the top sheet 1 in a longitudinal direction L2 from a direction of the bonded portion A2 toward a direction of the bonded portion A1 in the bonded portions A3 and A4, the first gather sheet 4 is provided to extend from the bonded portion A1 toward the central portion of the top sheet 1 in the longitudinal direction L2, and so that the first gather sheet 4 and the second gather sheet 5A are overlapped with each other, thereby forming the opening 6. Since a manner in which the wearer wears the absorbent pad for men P1 thus formed is the same as that in the preceding example, it will not be described herein.

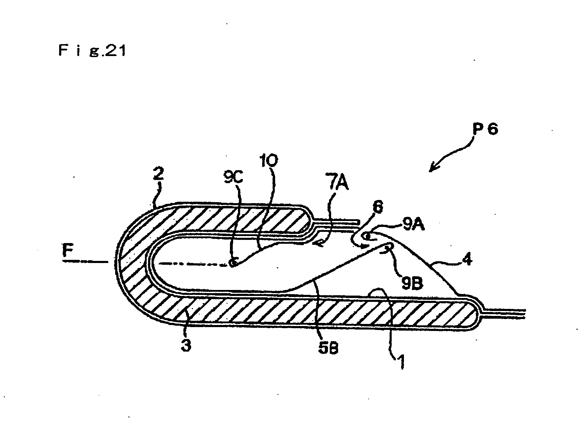

As shown in Fig. 21, an absorbent pad for men P6 may be formed as follows. The second gather sheet 5B is formed from the other end of front and rear ends of the top sheet 1, i.e., from the bonded portion A2 along the top sheet to extend toward the central portion of the top sheet 1. In addition, the first gather sheet 4 is formed to extend from one end thereof, i.e., from the bonded portion A1 toward the central portion of the top sheet 1 in the longitudinal direction L2. The opening 6 is formed so that the first gather sheet 4 and the second gather sheet 5 are overlapped with each other.

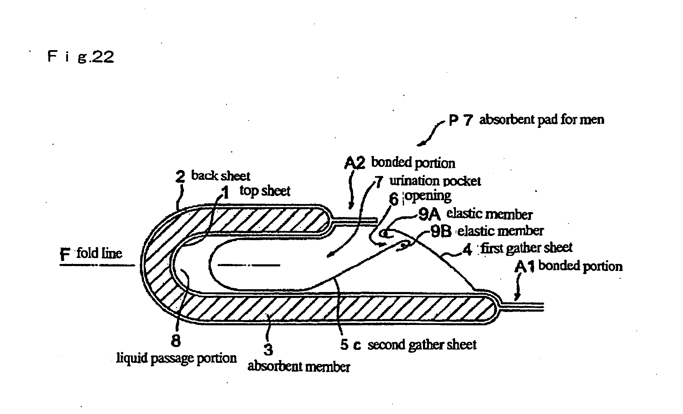

As shown in Fig. 22, as a modification of the example shown in Fig. 21, a gap is formed, as the liquid passage portion 8, between the top sheet 1 and a second gather sheet 5C without forming the second gather sheet 5C along the top sheet 1. Since the male genitals B are inserted between the second gather sheet 5 and the top sheet 1, the urine staying in the liquid passage portion 8 is sequentially absorbed and held by the absorbent ember 3 even if the wearer urinates with the wearer lying on his back. At this time, the elastic member 9B enables the second gather sheet 5 to be fit to the rear surface of the scrota, so that the urine does not leak outside.

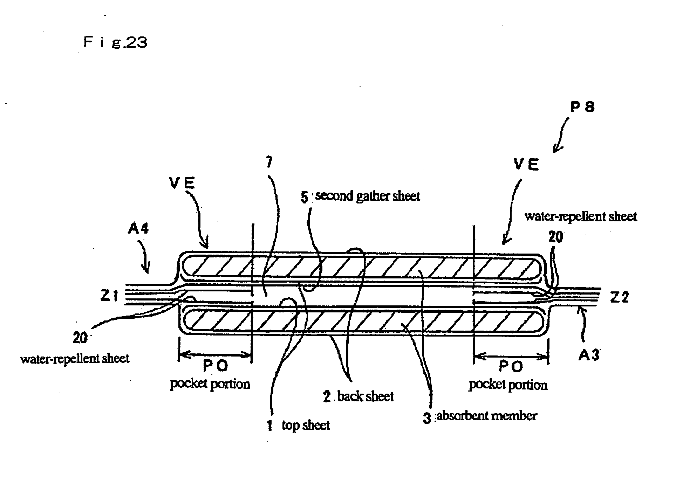

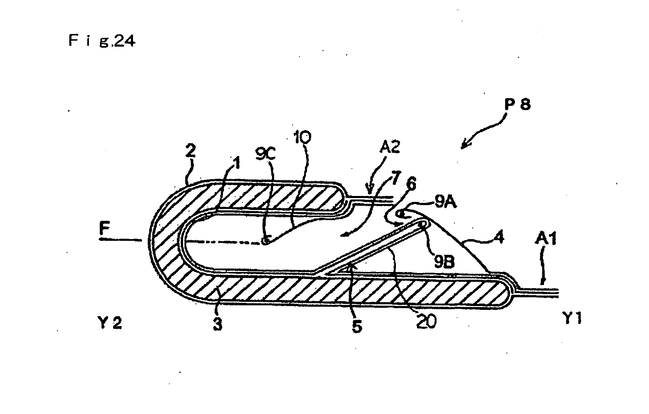

Yet another example of the absorbent pad for men will be described with reference to Figs. 23 and 24. An absorbent pad for men P8 in this example differs from that in the preceding example only in configurations of the bonded portions A3 and A4 and the both sides VEs of the absorbent member. Since the remaining configurations of the pad P8 are the same as those in the preceding example, they will not be described herein. The absorbent pad for men P8 in this example is configured so that liquid impermeable water-repellent sheets 20 are provided on the top sheet 1 so as to cover the both sides VEs of the absorbent member, respectively, peripheral edges of the back sheet 2 and the water-repellent sheets 20 are bonded to one another, thereby forming the bonded portions A1 to A4. More specifically, the both sides of the top sheet 1 are formed to be held between the absorbent member 3 and the back sheet 2, the water-repellent sheets 20 are provided to extend toward the sides of the back sheet 2 and bonded to the back sheet 2, thereby forming the bonded portions A3 and A4. Alternatively, the top sheet 1 may be configured to extend halfway along the bonded portions A3 and A4.

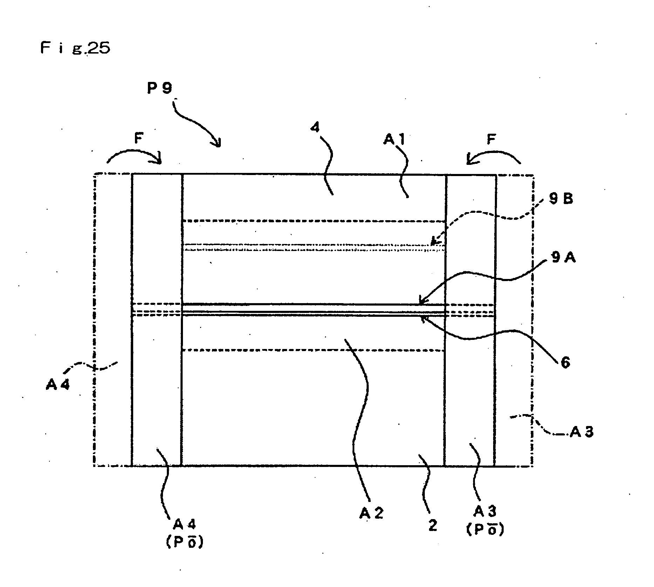

Further, as still another example of the absorbent pad for men, the bonded portions A3 and A4 may be folded back toward the opening 6 side as indicated by arrows F in place of providing the water-repellent sheets 20 on the top sheet 1 of the absorbent pad for men P9, as shown in Fig. 25. In this case, the hot melt adhesive or the like are applied to the bonded portions A3 and A4 to bond the bonded portions A3 and A4 to the back sheet 2 and the first gather sheet 4 on the opening 6 side. If so, the liquid impermeable back sheet 2 can prevent the urine staying in the pockets PO from leaking. Needless to say, not only the water-repellent sheets 20 are provided on the top sheet 1 of the absorbent pad for men P9 but also the bonded portions A3 and A4 may be folded back toward the opening 6 side. If so, the greater effect of preventing the leakage of urine can be exhibited.

While the tip end of the first gather sheet 4 is overlapped with that of the second gather sheet 5, the tip ends thereof may be located generally at the same position.

Furthermore, each of the absorbent pad for mens P4 to P9 is formed into a rectangle. However, each of the absorbent pad for mens P4 to P9 may be formed into a rectangle having corners notched. If so, when the wearer wears one of the absorbent pad for mens P4 to P9, the notched parts can improve the wearer's sense that the hip joint is fit to the pad P. Needless to say, the same advantages can be attained even if only corners of the rectangular absorbent member 3 are notched without notching those of the top sheet 1 and the back sheet 2.

Moreover, in a plurality of examples described so far, the water-repellent sheets (water-repellent strips) 20 are provided on the top sheet 1. However, the water-repellent sheets (water-repellent strips) 20 may be provided between the top sheet 1 and the absorbent member 3.

### <OTHERS>

Common matters to the above explained absorbent pads will be described below.

The top sheet 1 used in the present invention is a liquid permeable nonwoven fabric, a liquid permeable fabric, a liquid permeable porous plastic film or the like. Constituent fibers of the top sheet 1 are single fibers consisting of polypropylene, polyethylene, polyester, nylon or the like, composite fibers consisting of two or more components selected from a group consisting of polyester, polypropylene, polyethylene, nylon, and the like, or the like. The composite fibers consisting of polyester-polyester fibers, polyester-polyethylene fibers, or polypropylene-polyethylene fibers are preferably, but limited thereto, used in view of strength.

The back sheet 2 used in the present invention is a liquid impermeable film of polyethylene or the like, a material obtained by bonding the liquid impermeable film to a nonwoven fabric or fabric, or the like. It is more preferable to use a polyethylene film having many pores formed therein to the extent that liquid cannot be transmitted by the film, a moisture permeable film obtained by drawing a thermoplastic resin while adding filler thereto, or a composite sheet having the nonwoven fabric or fabric bonded to outside of one of these films, so as to prevent the wearer who wears the pad from feeling hot and stuffy. If so, excessive moisture within the absorbent pad is discharged to the outside of the pad in a gaseous state. Thanks to this, the wearer less frequently feels hot and stuffy and breaks out in a rash, whereby good moisture permeability and comfortable touch of the absorbent pad for men can be ensured.

The absorbent pad 3 used in the present invention consists of, but is limited thereto, flocculent pulp, high absorbent polymer (hereinafter, "SAP" in short), a hydrophilic sheet or the like. The absorbent member used in the present invention consists of a well-known absorbent material normally used for a conventional absorbent pad for men or the other absorbent articles.

That is, the absorbent member formed by wrapping up a single-layer or multilayer mat in which absorbent fibers of rayon or the like and SAPs are mixed together or mat layers consisting of absorbent fibers and having SAPs uniformly arranged between the layers in a hydrophilic sheet can be used.

As the flocculent pulp, powder obtained by defibrating a chemical pulp sheet or a mechanical pulp sheet using a pulverizing mill. As a raw material for the pulp, broadleaf tree, straw, bamboo, or kenaf as well as needle-leaf tree is available. Alternatively, waste paper pulp may be used. A utilization amount of this flocculent pulp depends on the intended absorbed member, e.g., whether a single absorbent member is used, a multilayered absorbent member is used, the absorbent member together with the other absorbent material is used. Normally, the amount of the flocculent pulp is 50 to 600 g/m² o50 to 400 g/m².

Examples of the SAP include a starch polymer, cellulose, and a synthetic polymer. Namely, a starch-acrylic acid (acrylate) graft copolymer, a saponified starch-ethyl acrylate graft copolymer, a saponified starch-methyl methacrylate graft copolymer, a saponified starch-acrylonitrile graft copolymer, a saponified starch-acrylamide graft copolymer, an acrylic acid (acrylate) copolymer, a polyethylene oxide cross-linked with acrylic acid, a cross-linker of sodium carboxymethylcellulose, a cross-linker of polyvinyl alcohol-maleic acid anhydride reactant, or the like can be used as the SAP.

To compress the absorbent member 3, the absorbent member 3 may be compressed into continuous surfaces so as to have a substantially uniform density over the entire absorbent member 3 using a roll having a smooth circumference. Alternatively, the absorbent member 3 may be compressed to have an embossed member in a pattern arrangement having partially different densities and guiding the urine or body fluid longitudinally and diagonally. If the absorbent member 3 is embossed, compressed parts and uncompressed parts may be either continuous or non-continuous.

As the water-repellent gather sheet used in the present invention, water-repellent finished nonwoven fabric or fabric, a liquid impermeable film of polyethylene or the like, a material obtained by bonding the liquid impermeable film to the nonwoven fabric or fabric, or the like can be used. If the liquid impermeable film is used, a polyethylene film having many pores formed therein to the extent that liquid molecules cannot be permeated or a moisture permeable film obtained by drawing a thermoplastic resin with filer added to the resin so as to prevent the wearer from feeling stuffy and hot is preferably used.

The liquid permeable gather sheet used in the present invention is, similarly to the top sheet 1, a liquid permeable nonwoven fabric, a liquid permeable fabric, a liquid permeable porous plastic film or the like. Constituent fibers of the top sheet 1 are single fibers consisting of polypropylene, polyethylene, polyester, nylon or the like, composite fibers consisting of two or more components selected from a group consisting of polyester, polypropylene, polyethylene, nylon, and the like, or the like. The composite fibers consisting of polyester-polyester fibers, polyester-polyethylene fibers, or polypropylene-polyethylene fibers are preferably, but limited thereto, used in view of strength.

As the water-repellent sheet 20 according to the present invention, water-repellent finished nonwoven fabric or fabric, a liquid impermeable film of polyethylene or the like, a material obtained by bonding the liquid impermeable film to the nonwoven fabric or fabric, or the like can be used. If the liquid impermeable film is used, a polyethylene film having many pores formed therein to the extent that liquid molecules cannot be permeated or a moisture permeable film obtained by drawing a thermoplastic resin with filer added to the resin so as to prevent the wearer from feeling stuffy and hot is preferably used.

If the nonwoven fabric or fabric is used, constituent fibers thereof are single fibers consisting of polypropylene, polyethylene, polyester, nylon or the like, composite fibers consisting of two or more components selected from a group consisting of polyester, polypropylene, polyethylene, nylon, and the like, or the like. The composite fibers consisting of polyester-polyester fibers, polyester-polyethylene fibers, or polypropylene-polyethylene fibers are preferably, but limited thereto, used in view of strength.

As the elastic member 9, a filament, corded, netted, or flat stretchable elastic member used in an ordinary absorbent pad for men or an ordinary absorbent article such as natural or synthetic rubber or urethane can be used. The number of the elastic members to be used may be one or two or more. The stretchable elastic member is arranged in an expanded state and fixedly bonded to the water-repellent material by using hot melt adhesive, water-soluble paste such as starch or CMC (carboxymethylcellulose), or high fluidity adhesive, or thermal, ultrasonic welding or the other welding.

The top sheet 1 and the gather sheet are bonded to each other on outer peripheral edges by the hot melt, water-soluble paste such as starch or CMC (carboxymethylcellulose), high fluidity adhesive, or by the thermal, ultrasonic welding or other welding.

## Claims

1. An absorbent pad for men comprising: a liquid permeable top sheet (1); a back sheet (2); an absorbent member (3) arranged between the top sheet (1) and the back sheet (2); and a pair of liquid impermeable first gather sheet (4) and liquid impermeable second gather sheet (5) facing each other on said top sheet (1), wherein
a liquid permeable auxiliary sheet (10) is provided to extend from a side of said second gather sheet (5) toward said first gather sheet (4) between said top sheet (1) and said gather sheets (4, 5), and wherein an opening (6') for inserting male genitals (B) is formed by the auxiliary sheet (10) and said first gather sheet (4),
**characterized in that**
an elastic member (9) is provided on a tip end (17T) of said auxiliary sheet (10), the tip end (17T) of said auxiliary sheet (10) is provided to enter below said first gather sheet (4), and the tip end (17T) of said auxiliary sheet (10) is at the same position as the tip end (TA) of said second gather sheet (5).

2. The absorbent pad for men according to claim 1, wherein said auxiliary sheet (10) is provided integrally with said top sheet (1).

3. The absorbent pad for men according to claim 1, wherein said auxiliary sheet (10) is provided separately from said top sheet (1).

4. The absorbent pad for men according to any one of claims 1 to 3, wherein said back sheet (2) is liquid impermeable.

5. The absorbent pad for men according to any one of claims 1 to 3, wherein said back sheet (2) is liquid permeable.

6. The absorbent pad for men according to any one of claims 1 to 5, wherein the absorbent member (3) is a rectangle with corners notched.

7. The absorbent pad for men according to any one of claims 1 to 6, wherein the opening (6') formed by a tip end (17T) of said auxiliary sheet (10) and a tip end (TA) of said first gather sheet (4) is unevenly distributed toward said first gather sheet (4).

## Patentansprüche

1. Saugeinlage für Männer, die umfasst: eine für Flüssigkeit durchlässige obere Lage (1); eine Gegenlage (2); ein Saugelement (3), das zwischen der oberen Lage (1) und der Gegenlage (2) angeordnet ist; und ein Paar aus einer für Flüssigkeit nicht durchlässigen ersten Sammellage (4) und einer für Flüssigkeit nicht durchlässigen zweiten Sammellage (5), die einander auf der oberen Lage (1) zugewandt sind, wobei
eine für Flüssigkeit durchlässige Hilfslage (10) vorgesehen ist, die sich zwischen der oberen Lage (1) und den Sammellagen (4, 5) von einer Seite der zweiten Sammellage (5) zu der ersten Sammellage (4) erstreckt, und wobei eine Öffnung (6') zum Einführen männlicher Genitalien (B) durch die Hilfslage (10) und die erste Sammellage (4) gebildet ist,
**dadurch gekennzeichnet, dass**
an einem spitzen Ende (17T) der Hilfslage (10) ein elastisches Element (9) vorgesehen ist, wobei das spitze Ende (17T) der Hilfslage (10) vorgesehen ist, um unter die erste Sammellage (4) einzudringen, und wobei sich das spitze Ende (17T) der Hilfslage (10) an derselben Position befindet wie das spitze Ende (TA) der zweiten Sammellage (5).

2. Saugeinlage für Männer nach Anspruch 1, wobei die Hilfslage (10) einteilig mit der oberen Lage (1) vorgesehen ist.

3. Saugeinlage für Männer nach Anspruch 1, wobei die Hilfslage (10) getrennt von der oberen Lage (1) vorgesehen ist.

4. Saugeinlage für Männer nach einem der Ansprüche 1 bis 3, wobei die Gegenlage (2) für Flüssigkeit nicht durchlässig ist.

5. Saugeinlage für Männer nach einem der Ansprüche 1 bis 3, wobei die Gegenlage (2) für Flüssigkeit durchlässig ist.

6. Saugeinlage für Männer nach einem der Ansprüche 1 bis 5, wobei das Saugelement (3) ein Rechteck mit gekerbten Ecken ist.

7. Saugeinlage für Männer nach einem der Ansprüche 1 bis 6, wobei die durch ein spitzes Ende (17T) der Hilfslage (10) und ein spitzes Ende (TA) der ersten Sammellage (4) gebildete Öffnung (6') in Richtung zu der ersten Sammellage (4) ungleichmäßig verteilt ist.

## Revendications

1. Protection absorbante pour hommes comprenant : une feuille supérieure perméable aux liquides (1) ; une feuille arrière (2) ; un élément absorbant (3) agencé entre la feuille supérieure (1) et la feuille arrière (2) ; et une paire de première feuille de liaison imperméable aux liquides (4) et de deuxième feuille de liaison imperméable aux liquides (5) se faisant face mutuellement sur la feuille supérieure (1), où
une feuille auxiliaire perméable aux liquides (10) est prévue de manière à s'étendre depuis un côté de ladite deuxième feuille de liaison (5) vers ladite première feuille de liaison (4) entre ladite feuille supérieure (1) et lesdites feuilles de liaison (4, 5), et où une ouverture (6') permettant d'insérer les organes génitaux masculins (B) est formée par la feuille auxiliaire (10) et ladite première feuille de liaison (4),
**caractérisée en ce que**
un élément élastique (9) est prévu sur une extrémité de pointe (17T) de ladite feuille auxiliaire (10), l'extrémité de pointe (17T) de ladite feuille auxiliaire (10) est prévue pour entrer au-dessous de ladite première feuille de liaison (4), et l'extrémité de la pointe (17T) de ladite feuille auxiliaire (10) se trouve à la même position que l'extrémité de pointe (TA) de ladite deuxième feuille de liaison (5).

2. Protection absorbante pour hommes selon la revendication 1, dans laquelle
ladite feuille auxiliaire (10) est prévue de manière solidaire avec ladite feuille supérieure (1).

3. Protection absorbante pour hommes selon la revendication 1, dans laquelle
ladite feuille auxiliaire (10) est prévue séparément de ladite feuille supérieure (1).

4. Protection absorbante pour hommes selon l'une quelconque des revendications 1 à 3, dans laquelle ladite feuille arrière (2) est imperméable aux liquides.

5. Protection absorbante pour hommes selon l'une quelconque des revendications 1 à 3, dans laquelle ladite feuille arrière (2) est perméable aux liquides.

6. Protection absorbante pour hommes selon l'une quelconque des revendications 1 à 5, dans laquelle l'élément absorbant (3) est un rectangle à coins rentrés.

7. Protection absorbante pour hommes selon l'une quelconque des revendications 1 à 6, dans laquelle l'ouverture (6') formée par une extrémité de pointe (17T) de ladite feuille auxiliaire (10) et une extrémité de pointe (TA) de ladite première feuille de liaison (4) est inégalement répartie vers ladite première feuille de liaison (4).
